# EUROPÄISCHE PATENTANMELDUNG

(11) **EP 2 883 864 A1**
(43) Veröffentlichungstag der Anmeldung: **17.06.2015**
(21) Anmeldenummer: 13196585.7
(22) Anmeldetag: 11.12.2013
(51) Int. Cl.: C07C 209/72

(54) **Verfahren zur hydrierung aromatischer verbindungen**

(71) Anmelder: BASF SE, 67056 Ludwigshafen (DE)
(72) Erfinder: Schaack, Bernd Bastian, 64625 Bensheim (DE); Bock, Martin, 67063 Ludwigshafen (DE); Dahmen, Kirsten, 67098 Bad Dürkheim (DE)
(74) Vertreter: Steinbusch, Daniel

(57) **Zusammenfassung**

Die vorliegende Erfindung betrifft ein Verfahren zur Hydrierung aromatischer Verbindungen mit Wasserstoff in Gegenwart eines Katalysators, wobei der Katalysator Ruthenium auf einem Zirkoniumoxid-Trägermaterial enthält, sowie die Verwendung eines Katalysators enthaltend Ruthenium auf einem Zirkoniumoxid-Trägermaterial zur Hydrierung von aromatischen Verbindungen.

## Beschreibung

Die vorliegende Erfindung betrifft ein Verfahren zur Hydrierung aromatischer Verbindungen mit Wasserstoff in Gegenwart eines Katalysators, wobei der Katalysator Ruthenium auf einem Zirkoniumoxid-Trägermaterial enthält, sowie die Verwendung eines Katalysators enthaltend Ruthenium auf einem Zirkoniumoxid-Trägermaterial zur Hydrierung von aromatischen Verbindungen.

Verfahren zur Hydrierung organischer Verbindungen, insbesondere zur Hydrierung von aromatischen Aminen zu den entsprechenden Cyclohexan-Derivaten sind aus dem Stand der Technik bereits bekannt.

WO 2009/153123 A1 offenbart ein kontinuierliches Verfahren und einen Reaktor zur Hydrierung organischer Verbindungen in einem mehrphasigen System in Gegenwart eines homogenen oder heterogenen Katalysators, wobei das Verfahren in zwei Stufen durchgeführt wird. Als mögliche Katalysatoren gemäß diesem Dokument werden heterogene Katalysatoren, beispielsweise enthaltend Edelmetalle wie Platin, Palladium, Ruthenium und Rhodium oder andere Übergangsmetalle, wie Molybdän, Wolfram und Chrom offenbart. Diese heterogenen Katalysatoren können auf Trägermaterialien vorliegen. Entsprechende Trägermaterialien sind beispielsweise Kohlenstoff, Aluminiumoxid, Siliciumdioxid, Zirkoniumdioxid, Zeolithe, Alumosilikate oder Mischungen dieser Trägermaterialien. In Beispiel 1 wurde eine MDA-Schmelze in Gegenwart eines suspendierten Ru-(IV)-oxidhydrat-Katalysators hydriert. Ausführungsbeispiele zur Hydrierung von MDA in Gegenwart von Ru, geträgert auf Zirkoniumoxid, sind in der Anmeldung nicht enthalten. Als Substrate werden in diesem Verfahren bevorzugt aromatische Verbindungen enthaltend Amino-Substituenten eingesetzt, beispielsweise Polymer-MDA, Anilin, 2,4-Diaminotoluol, 2,6-Diaminotoluol, o-Phenylendiamin, etc. Die heterogenen Katalysatoren werden in Suspension eingesetzt.

DE 19533718 A1 offenbart ein Verfahren zur Hydrierung von aromatischen Verbindungen, in denen mindestens eine Aminogruppe an einen aromatischen Kern gebunden ist. Dazu kann ein heterogener Katalysator enthaltend Ruthenium und gegebenenfalls mindestens ein Metall der I-, VII- oder VIII-Nebengruppe enthalten ist. Als Trägermaterial wird beispielsweise Aluminiumoxid, Siliciumdioxid, Titandioxid oder Zirkoniumdioxid, vorzugsweise Aluminiumdioxid oder Zirkoniumdioxid, eingesetzt. Als Beispiel angegeben wird lediglich ein Katalysator enthaltend Ruthenium auf dem Trägermaterial Aluminiumoxid, nicht jedoch Zirkoniumoxid.

EP 1337331 B1 offenbart ein Verfahren zur katalytischen Hydrierung von aromatischen oder heteroaromatischen Aminen, dadurch gekennzeichnet, dass das Aktivmetall Ruthenium ist und der Katalysator mindestens ein weiteres Metall der I-, VII-, oder VIII-Nebengruppe enthält und diese auf einem Trägermaterial aufgebracht sind, das eine BET(N₂)-Oberfläche von weniger als 10 m²/g aufweist. Als aromatische Verbindungen werden beispielsweise 4,4'-MDA und Isomeren hiervon eingesetzt.

In EP 1366812 B1 wird ein Verfahren zur Hydrierung eines aromatischen Amins offenbart, das in Gegenwart des Aktivmetalls Ruthenium auf einem Trägermaterial durchgeführt wird. Das Verfahren ist dadurch gekennzeichnet, dass die BET-Oberfläche des verwendeten Trägermaterials im Bereich von größer 30 m²/g bis kleiner 70 m²/g liegt. Als Trägermaterialien werden unter anderem Aluminiumoxid, Siliciumoxid, Titanoxid und Zirkoniumoxid offenbart. In den Beispielen wird lediglich Aluminiumoxid als Trägermaterial eingesetzt, nicht jedoch Zirkoniumoxid.

WO 2011/003899 A1 offenbart ein Verfahren zur Hydrierung organischer Verbindungen, beispielsweise aromatischer Verbindungen. Dazu kann ein heterogener Katalysator enthaltend Edelmetalle, wie Platin, Palladium, Ruthenium, Osmium, Iridium und Rhodium oder andere Übergangsmetalle eingesetzt werden. Als Trägermaterialien werden beispielsweise Aluminiumoxid, Siliciumdioxid, Titandioxid und Aktivkohle genannt.

WO 2011/033104 A1 offenbart eine Zusammensetzung, die mindestens ein Epoxidharz und eine Mischung enthaltend Stereoisomere von Diaminomethylcyclohexan enthält, und ein Verfahren zur Herstellung dieser Zusammensetzung. Die Herstellung erfolgt dabei durch Hydrierung der entsprechenden aromatischen Verbindungen in Gegenwart eines Katalysators beispielsweise enthaltend Rhodium, Ruthenium, Palladium, Platin oder Nickel. Als Trägermaterialien können Aluminiumoxid, Siliciumoxid und Kohlenstoff eingesetzt werden. Die Hydrierung kann kontinuierlich oder diskontinuierlich, jeweils in Suspension, durchgeführt werden.

EP-0-796 839 A1 offenbart ein Verfahren zur kontinuierlichen Herstellung eines Gemisches aus Aminomethylcyclohexanen und Diaminomethylcyclohexanen durch katalytische Hydrierung von Aminotoluolen und Diaminotoluolen mit Wasserstoff unter Verwendung eines Festbettkatalysators aus einem mit Verbindungen von Ruthenium, Seltenerdmetallen, Mangan sowie Alkalimetallhydroxiden oder Erdalkalimetallhydroxiden behandelten Aluminiumoxid-Trägermaterial.

EP-0-443 344 A2 offenbart ein Verfahren zur Herstellung von 1-Methyl-2,6-cyclohexandiamin. Dazu wird ein Suspensionskatalysator enthaltend Rhodium auf Aluminiumoxid eingesetzt und 2,6-Toluoldiamin mit Wasserstoff hydriert.

Die aus dem Stand der Technik bekannten Verfahren zur Hydrierung aromatischer Verbindungen, um die entsprechenden Cyclohexanderivate zu erhalten, weisen bezüglich der Deaktivierungsneigung der eingesetzten Katalysatoren noch Verbesserungspotential auf. Bei den Verfahren aus dem Stand der Technik erleidet der eingesetzte Katalysator nach relativ kurzer Zeit eine Deaktivierung, d. h., der erzielte Umsatz an gewünschtem Produkt sinkt. Des Weiteren tritt bei den aus dem Stand der Technik bekannten Verfahren, in denen der Katalysator im Festbett eingesetzt wird, häufig das Problem des Sinterns der Katalysatoren und/oder der Koksbildung auf dem Katalysator auf.

Aufgabe der vorliegenden Erfindung ist es daher, ein entsprechendes Verfahren zur Hydrierung von aromatischen Aminen bereitzustellen, in dem ein Katalysator eingesetzt wird, der über einen langen Zeitraum eine besonders hohe Aktivität aufweist, so dass über einen langen Zeitraum ein hoher Umsatz bei hoher Selektivität erzielt werden kann. Des Weiteren soll erfindungsgemäß im Festbett oder in Suspension gearbeitet werden können, ohne dass die aus Verfahren des Standes der Technik bekannten Nachteile auftreten, beispielsweise Koksbildung bzw. Sinterung des eingesetzten Katalysators bei einer Festbettreaktion.

Diese Aufgaben werden gelöst durch das erfindungsgemäße Verfahren zur Hydrierung aromatischer Verbindungen mit Wasserstoff in Gegenwart eines Katalysators, wobei der Katalysator Ruthenium auf einem Zirkoniumoxid-Trägermaterial enthält. Die Aufgaben werden des Weiteren gelöst durch die Verwendung eines Katalysators enthaltend Ruthenium auf einem Zirkoniumoxid-Trägermaterial zur Hydrierung aromatischer Verbindungen.

Die vorliegende Erfindung zeichnet sich dadurch aus, dass ein Katalysator im Festbett oder in Suspension eingesetzt wird, der Ruthenium auf einem Zirkoniumoxid-Trägermaterial enthält.

Durch das erfindungsgemäße Verfahren können im Allgemeinen alle aromatischen Verbindungen mit Wasserstoff zu den entsprechenden Hydrierprodukten umgesetzt werden.

Als aromatische Verbindungen für die Hydrierung nach dem erfindungsgemäßen Verfahren kommen bevorzugt in Betracht:

Aromatische Monoamine, wie Anilin, isomere Toluidine, isomere Xylidine, 1- bzw. 2-Aminonaphthalin, Benzidin und substituierte Benzidine, aromatische Diamine, wie isomere Phenylendiamine (o-Phenylendiamin, m-Phenylendiamin, p-Phenylendiamin), isomere Toluylendiamine, wie 2,4-Diaminotoluol, 2,6-Diaminotoluol, 2,3-Diaminotoluol, 2,5-Diaminotoluol, 3,4-Diaminotoluol, 3,5-Diaminotoluol, isomere Diaminonaphthalene, wie 1,5-Diaminonaphthalin, Meta-Xylendiamin (MXDA), Bis-(4-amino-3-methylphenyl)-methan, und Bis-(4-amino-3,5-dimethylphenyl)-methan, aromatische Mono- und Dicarbonsäuren und deren Ester, wie Benzoesäure, isomere Phthalsäuren sowie deren Ester, bevorzugt deren Methylester, aromatische Aminocarbonsäuren und deren Ester, wie Anthranilsäure, aromatische Alkohole, wie Phenol und Bisphenol A, sowie aromatische Kohlenwasserstoffe, wie Benzol, Toluol, Ethylbenzol, isomere Xylole, Inden, Tetralin und Naphthalin.

In einer besonders bevorzugten Ausführungsform betrifft die vorliegende Erfindung das erfindungsgemäße Verfahren, wobei die organische Verbindung ein aromatisches Amin ist, weiter bevorzugt ausgewählt aus der Gruppe bestehend aus Anilin, 2,4-Diaminotoluol (2,4-TDA), 2,6-Diaminotoluol (2,6-TDA), 2,3-Diaminotoluol, 2,5-Diaminotoluol, 3,4-Diaminiotoluol, 3,5-Diaminotoluol, o-Phenylendiamin, m-Phenylendiamin, p-Phenylendiamin, m-Xylendiamin (MXDA), Bis-(4-amino-3-methylphenyl)-methan, Bis-(4-amino-3,5-dimethylphenyl)-methan und Mischungen davon.

Erfindungsgemäß einsetzbare Mischungen können die oben genannten Verbindungen und/oder deren Isomere im Allgemeinen in jeder möglichen Zusammensetzung enthalten.

Ganz besonders bevorzugt wird in dem erfindungsgemäßen Verfahren eine Mischung der Isomeren von Toluoldiamin (TDA), bevorzugt eine Mischung enthaltend 10 bis 30 Gew.-%, besonders bevorzugt 15 bis 25 Gew.-%, beispielsweise 20 Gew.-%, 2,6-TDA und 70 bis 90 Gew.-%, besonders bevorzugt 75 bis 85 Gew.-%, beispielsweise 80 Gew.-%, 2,4-TDA, wobei die Summe der Mengen an 2,6-TDA und 2,4-TDA jeweils 100 Gew.-% ergibt, eingesetzt. Bei dieser besonders bevorzugten Ausführungsform wird als Produkt des erfindungsgemäßen Verfahrens eine Mischung der Isomeren von Methyldiaminocyclohexan (MDACH) erhalten.

Die vorliegende Erfindung betrifft daher bevorzugt das erfindungsgemäße Verfahren, wobei die organische Verbindung ein Gemisch aus 2,4- und 2,6-Diaminotoluol ist, wobei das 2,4-Diaminotoluol in einem Anteil von 70 bis 90% und das 2,6-Diaminotoluol in einem Anteil von 10-30% vorliegt.

Das erfindungsgemäße Verfahren kann im Allgemeinen kontinuierlich oder diskontinuierlich durchgeführt werden. In einer bevorzugten Ausführungsform betrifft die vorliegende Erfindung das erfindungsgemäße Verfahren, wobei es kontinuierlich durchgeführt wird.

Bei der diskontinuierlichen Reaktionsführung kann die Hydrierung beispielsweise in einem Rührkessel bzw. Rührautoklaven, einem Schlaufenreaktor, einem Strahlschlaufenreaktor, einer Blasensäule oder einem Festbettreaktor mit Umpumpkreislauf durchgeführt werden. Bevorzugt wird die diskontinuierliche Hydrierung in einem Rührkessel bzw. Rührautoklaven durchgeführt.

Bei der kontinuierlichen Reaktionsführung wird die Hydrierung üblicherweise in einem kontinuierlich betriebenen Rührkesselreaktor, einem kontinuierlich betriebenen Schlaufenreaktor, einem kontinuierlich betriebenen Strahlschlaufenreaktor, einer kontinuierlich betriebenen Blasensäule oder einem kontinuierlich betriebenen Festbettreaktor mit Umpumpkreislauf oder einer Rührkesselkaskade durchgeführt. Bevorzugt wird die diskontinuierliche Hydrierung in einem Rührkessel bzw. Rührautoklaven durchgeführt.

Vorzugsweise führt man das Verfahren in Rieselreaktoren oder in gefluteter Fahrweise nach der Festbettfahrweise durch, beispielsweise gemäß der WO 2008/015135 A1. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung am Katalysatorfließbett und am Katalysatorfestbett sind aus dem Stand der Technik bekannt, z.B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM. Geeignete Reaktoren für die Durchführung des erfindungsgemäßen Verfahrens in Suspensionsfahrweise sind dem Fachmann an sich bekannt, beispielsweise Rührkessel oder Blasensäule. Erfindungsgemäß ist es auch möglich, dass in einer Kaskade mehrerer hintereinander geschalteter Suspensionsreaktoren gearbeitet wird, beispielsweise in einer Rührkesselkaskade oder einer Blasensäulenkaskade, beispielsweise mit jeweils mindestens drei entsprechenden, hintereinandergeschalteten Reaktoren.

Um einen vollständigen Umsatz zu erzielen, kann eine Nachreaktion des Hydrieraustrags erfolgen. Dazu kann der Hydrieraustrag im Anschluss an das Hydrierverfahren in der Gasphase oder in der Flüssigphase im geraden oder umgepumpten Durchgang, durch einen oder mehrere nachgeschaltete Reaktoren geleitet werden. Der Reaktor kann bei Flüssigphasenhydrierung in Rieselfahrweise betrieben oder geflutet betrieben werden. Der Reaktor ist mit dem erfindungsgemäßen oder mit einem anderen, dem Fachmann bekannten, Katalysator befüllt.

Das erfindungsgemäße Verfahren wird im Allgemeinen bei einem Druck von 50 bis 500 bar durchgeführt, vorzugsweise bei einem Druck von 100 bis 300 bar. Da das erfindungsgemäße Verfahren besonders bevorzugt ohne Zugabe eines weiteren Gases neben Wasserstoff durchgeführt wird, wird der Verfahrensdruck bevorzugt durch den Wasserstoff-Partialdruck bestimmt. Die vorliegende Erfindung betrifft daher insbesondere bevorzugt das erfindungsgemäße Verfahren, wobei es bei einem Wasserstoffdruck von 50 bis 500 bar, bevorzugt 100 bis 300 bar, durchgeführt wird.

Erfindungsgemäß wird das Verfahren bei einer Temperatur von 30 bis 280 °C durchgeführt, vorzugsweise 50 bis 220 °C, besonders bevorzugt 70 bis 190 °C.

Die vorliegende Erfindung betrifft daher bevorzugt das erfindungsgemäße Verfahren, wobei es bei einer Temperatur von 30 bis 280 °C vorzugsweise 50 bis 220 °C, besonders bevorzugt 70 bis 190 °C. durchgeführt wird.

In dem erfindungsgemäßen Verfahren wird Wasserstoff als Hydriermittel eingesetzt.

Der als Hydriermittel eingesetzte Wasserstoff wird in einer bevorzugten Ausführungsform im Überschuss bezogen auf die zu hydrierende Verbindung eingesetzt. Beispielsweise wird Wasserstoff als Hydriermittel in einem 1,01 bis 10-fachen, bevorzugt 1,05 bis 10-fachen, weiter bevorzugt 1- bis 10-fachen, besonders bevorzugt 1,01- bis 5-fachen, beispielsweise 1,1- bis 5-fachen, stöchiometrischen Überschuss eingesetzt. In einer Ausführungsform kann der eingesetzte Wasserstoff als Kreisgas in die Reaktion zurückgeführt werden.

In einer bevorzugten Ausführungsform des erfindungsgemäßen Verfahrens wird technisch reiner Wasserstoff eingesetzt. Im Rahmen der vorliegenden Erfindung wird unter "technisch rein" ein Gehalt an Wasserstoff von mindestens 99,0 Gew.-%, bevorzugt mindestens 99,5 Gew.-%, verstanden.

In einer weiteren erfindungsgemäßen Ausführungsform kann der Wasserstoff auch in Form eines Wasserstoff enthaltenden Gases eingesetzt werden. Möglich sind hierbei beispielsweise Mischungen enthaltend Gase und Inertgase wie Stickstoff, Helium, Neon, Argon und/oder Kohlendioxid. Als Wasserstoff enthaltende Gase können beispielsweise Reformerabgase, Raffineriegase usw. verwendet werden. Diese Wasserstoff enthaltenden Gase weisen einen Wasserstoffgehalt von beispielsweise 10 bis 100 Gew.-%, bevorzugt 50 bis 100 Gew.-%, auf.

Das erfindungsgemäße Verfahren kann im Allgemeinen in Gegenwart oder in Abwesenheit wenigstens eines Lösungsmittels durchgeführt werden. Besonders bevorzugt wird das Verfahren in einem organischen Lösungsmittel durchgeführt. In einer weiteren bevorzugten Ausführungsform wird das erfindungsgemäße Verfahren in Substanz, d. h. als Schmelze in Abwesenheit eines Lösungsmittels, durchgeführt.

Der Einsatz von Lösungsmitteln ist beispielsweise vorteilhaft, wenn die organische Verbindung als Feststoff vorliegt und sich nicht oder nur unter großem Aufwand als Schmelze handhaben und fördern lässt. Geeignete Lösungsmittel sind beispielsweise ausgewählt aus der Gruppe bestehend aus Alkoholen, wie Isopropanol, Isobutanol oder t-Butanol, Ethern, wie Diethylether, Glykoldimethylether (Diglyme), Glykoldipropylether (Proglyme), Dioxan oder Tetrahydrofuran, und Mischungen davon. In einer weiteren erfindungsgemäßen Ausführungsform werden das bei der Reaktion entstehende Endprodukt Methyldiaminocyclohexan oder die gebildeten Leichtsieder, zum Beispiel Methylcyclohexylamin, als Lösungsmittel eingesetzt.

Wird das erfindungsgemäße Verfahren in Gegenwart eines Lösungsmittels durchgeführt, so wird dieses im Allgemeinen in einer solchen Menge eingesetzt, so dass eine 10 bis 50 gew.-%ige, bevorzugt 15 bis 40 gew.-%ige, besonders bevorzugt 20 bis 30 gew.-%ige Lösung der zur Hydrierung vorgesehenen organischen Verbindungen vorliegt.

Erfindungsgemäß wird als Katalysator Ruthenium auf einem Zirkoniumoxid-Trägermaterial eingesetzt.

Entsprechende Katalysatoren können durch bekannte Verfahren wie Imprägnierung bzw. Tränkung, beispielsweise beschrieben in A. B. Stiles, Catalyst Manufacture-Laboratory and Commerical Preparations, Marcel Dekker, New York, 1983, oder Aus- bzw. Auffällung, beispielsweise beschrieben in EP 1 106 600, Seite 4, und A. B. Stiles, Catalyst Manufacture, Marcel Dekker Inc., 1983, Seite 15, hergestellt werden.

Zur Herstellung der erfindungsgemäß einzusetzenden Katalysatoren kann so vorgegangen werden, dass auf das Trägermaterial Zirkoniumoxid in Form von Strangpresslingen, Pillen oder Kugeln, beispielsweise mit Durchmessern von etwa 1,5 bis 10 mm, geeignete Verbindungen des Rutheniums, beispielsweise Rutheniumsalze, aufgebracht werden. Anschließend wird im Allgemeinen bei einer Temperatur von 80 bis 180 °C, beispielsweise 120 °C, getrocknet und bei einer Temperatur von 180 bis 450 °C, beispielsweise 180 °C, kalziniert, beide Schritte können auch simultan erfolgen. Für das Aufbringen geeignete Rutheniumsalze sind beispielsweise ausgewählt aus der Gruppe bestehend aus Ruthenium-acetat, -acetylacetonat, -chlorid, -nitrosylnitrat und Mischungen davon.

Ein entsprechend hergestellter Katalysator steht nach dem Trocknen grundsätzlich für den erfindungsgemäßen Einsatz zur Verfügung. In bevorzugter Weise wird er jedoch vor seinem Einsatz, besonders bevorzugt nach Anordnung in dem für die erfindungsgemäße Hydrierung vorgesehenen Reaktor, durch Behandlung mit Wasserstoff bei einer Temperatur von beispielsweise 150 bis 400 °C, aktiviert.

Auf dem erfindungsgemäß eingesetzten Katalysator liegt Ruthenium bevorzugt in einer Gesamtmenge von 0,05 bis 15 Gew.-%, bevorzugt 0,05 bis 12 Gew.-%, besonders bevorzugt 0,1 bis 11 Gew.-%, jeweils bezogen auf das Gesamtgewicht des Katalysators, vor.

Das Trägermaterial Zirkoniumoxid (ZrO₂) liegt erfindungsgemäß bevorzugt in monokliner, tetragonaler, kubischer, amorpher Phase oder einer Mischphase dieser Modifikationen, besonders bevorzugt in monokliner, tetragonaler oder einer Mischphase dieser Modifikationen, vor.

Die vorliegende Erfindung betrifft daher bevorzugt das erfindungsgemäße Verfahren, wobei das Zirkoniumoxid-Trägermaterial in monokliner, tetragonaler, kubischer, amorpher Phase oder einer Mischphase dieser Modifikationen, besonders bevorzugt in monokliner, tetragonaler oder einer Mischphase dieser Modifikationen vorliegt.

Erfindungsgemäß bevorzugt weist das Zirkoniumoxid-Trägermaterial, bevorzugt vor Aufbringen des Rutheniums, eine BET-Oberfläche von 30 bis 300 m²/g, bevorzugt 35 bis 250 m²/g, besonders bevorzugt 50 bis 90 m²/g, auf, jeweils bestimmt durch Stickstoffsorption nach DIN 66131.

Erfindungsgemäß bevorzugt weist der erfindungsgemäß eingesetzte Katalysator eine BET-Oberfläche von 30 bis 300 m²/g, bevorzugt 50 bis 90 m²/g, ein Porenvolumen von 0,1 bis 1 cm³/g, bevorzugt 0,1 bis 0,9 cm³/g, und eine Stampfdichte von 500 bis 2000 kg/m³, bevorzugt 700 bis 1750 kg/m³, auf.

Erfindungsgemäß bevorzugt weist das Zirkoniumoxid-Trägermaterial, bevorzugt vor Aufbringen des Rutheniums, ein Porenvolumen von 0,1 bis 1 cm³/g, bevorzugt 0,1 bis 0,9 cm³/g, auf, jeweils bestimmt durch Quecksilberporosimetrie nach DIN 66133.

Wird das erfindungsgemäße Verfahren im Festbett durchgeführt, so weist das Zirkoniumoxid-Trägermaterial, bevorzugt vor Aufbringen des Rutheniums, ein Porenvolumen von 0,1 bis 1 cm³/g, bevorzugt 0,1 bis 0,6 cm³/g, besonders bevorzugt 0,1 bis 0,5 cm³/g auf, jeweils bestimmt durch Quecksilberporosimetrie nach DIN 66133.

Wird das erfindungsgemäße Verfahren in Suspension durchgeführt, so weist das Zirkoniumoxid-Trägermaterial, bevorzugt vor Aufbringen des Rutheniums, ein Porenvolumen von 0,1 bis 1 cm³/g, bevorzugt 0,5 bis 1,0 cm³/g, besonders bevorzugt 0,7 bis 0,9 cm³/g auf, jeweils bestimmt durch Quecksilberporosimetrie nach DIN 66133.

Erfindungsgemäß bevorzugt weist das Zirkoniumoxid-Trägermaterial, bevorzugt vor Aufbringen des Rutheniums, eine Stampfdichte von 500 bis 2000 kg/m³, bevorzugt 600 bis 1800 kg/m³, besonders bevorzugt 700 bis 1750 kg/m³, auf , jeweils bestimmt in einem Stampfvolumeter STAV2003 der Firma JEL, wobei 2000 mal gestampft wurde.

Erfindungsgemäß bevorzugt weist das Zirkonoxid-Trägermaterial eine BET-Oberfläche von 30 bis 300 m²/g, bevorzugt 50 bis 90 m²/g, jeweils bestimmt durch Stickstoffsorption nach DIN 66131, ein Porenvolumen von 0,1 bis 1 cm³/g, bevorzugt 0,1 bis 0,9 cm³/g, jeweils bestimmt durch Quecksilberporosimetrie nach DIN 66133, und eine Stampfdichte von 500 bis 2000 kg/m³, bevorzugt 700 bis 1750 kg/m³, jeweils bestimmt in einem Stampfvolumeter STAV2003 der Firma JEL, wobei 2000 mal gestampft wurde, auf.

Erfindungsgemäß besonders bevorzugt weist das Zirkoniumoxid-Trägermaterial eine monokline oder tetragonale Modifikation (bzw. ein Gemisch beider), eine BET-Oberfläche von 30 bis 300 m²/g, bevorzugt 50 bis 90 m²/g, jeweils bestimmt durch Stickstoffsorption nach DIN 66131, ein Porenvolumen von 0,1 bis 1 cm³/g, bevorzugt 0,1 bis 0,9 cm³/g, jeweils bestimmt durch Quecksilberporosimetrie nach DIN 66133, und eine Stampfdichte von 500 bis 2000 kg/m³, bevorzugt 700 bis 1750 kg/m³, jeweils bestimmt in einem Stampfvolumeter STAV2003 der Firma JEL, wobei 2000 mal gestampft wurde, auf.

Das Zirkoniumoxid-Trägermaterial des im Festbett eingesetzten Katalysators weist eine Porengrößenverteilung auf, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

Die vorliegende Erfindung betrifft daher bevorzugt das erfindungsgemäße Verfahren, wobei das Zirkoniumoxid-Trägermaterial des im Festbett eingesetzten Katalysators eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

Das Zirkoniumoxid-Trägermaterial des in Suspension eingesetzten Katalysators weist eine Porengrößenverteilung auf, bei der mehr als 40% der vorliegenden Poren durch Makroporen mit einem Durchmesser von >50 nm, und der Rest zu 100% durch Mesoporen mit einem Durchmesser von 2 nm bis 50 nm gebildet werden.

Die vorliegende Erfindung betrifft daher bevorzugt das erfindungsgemäße Verfahren, wobei das Zirkoniumoxid-Trägermaterial des in Suspension eingesetzten Katalysators eine Porengrößenverteilung aufweist, bei der mehr als 40% der vorliegenden Poren durch Makroporen mit einem Durchmesser von >50 nm, und der Rest zu 100% durch Mesoporen mit einem Durchmesser von 2 nm bis 50 nm gebildet werden.

In dem erfindungsgemäßen Verfahren wird im Allgemeinen eine Katalysatorbelastung von 0,01 bis 2 kg, bevorzugt 0,05 bis 1 kg, besonders bevorzugt 0,10 bis 0,6 kg aromatische, zu hydrierende Verbindung pro Liter Katalysator pro Stunde eingestellt. Eine gegebenenfalls während des erfindungsgemäßen Verfahrens stattfindende geringe Veränderung des erzielten Anteils an gewünschten Produkt durch eine sich gegebenenfalls verändernde Aktivität des Katalysators im Laufe besonders langer Reaktionsperioden kann durch ein geringes Nachstellen der Reaktionstemperatur oder der anderen Parameter ausgeglichen werden. Die sich gegebenenfalls verändernden Anteile an gewünschtem Produkt können durch die Analytik des Reaktionsgemisches verfolgt werden. Diese Analytik kann durch dem Fachmann bekannte Methoden erfolgen, beispielsweise Gaschromatographie (GC).

Die erfindungsgemäß erhaltenen Hydriergemische können nach dem erfindungsgemäßen Verfahren gereinigt werden, beispielsweise durch Destillation. Gegebenenfalls im Reaktionsaustrag vorhandener Katalysator kann vor der Destillation entfernt werden, beispielsweise durch eine Fest-Flüssig-Trennung, wie Filtration, Sedimentation oder Zentrifugation. Lösungsmittel und nicht umgesetzte Ausgangsstoffe können in das Verfahren zurückgeführt werden.
Die erfindungsgemäß gewünschten Produkte werden nach erfolgreicher Aufarbeitung, beispielsweise durch Destillation, in einer Reinheit von mindestens 99 Gew.-% erhalten. In dieser Reinheit sind die genannten Verbindungen in der Regel für alle weiterverarbeitenden Prozesse einsetzbar.

Die vorliegende Erfindung betrifft auch die Verwendung eines Katalysators enthaltend Ruthenium auf einem Zirkoniumoxid-Trägermaterial zur Hydrierung aromatischer Verbindungen. Bezüglich der einzelnen Merkmale und der bevorzugten Ausführungsformen dieser erfindungsgemäßen Verwendung gilt das bezüglich des erfindungsgemäßen Verfahrens Gesagte entsprechend.

Das erfindungsgemäße Verfahren und dessen Vorteile werden durch die folgenden Beispiele näher erläutert.

### Beispiele

### Beispiel 1

Verschiedene katalytisch aktive Metalle auf Al₂O₃ als Träger wurden in dem erfindungsgemäßen Verfahren in der Hydrierung von Toluoldiamin (TDA) zu Methyldiaminocyclohexan (MDACH) getestet und der Umsatz und die Selektivität gemessen. Die erfindungsgemäßen Reaktionsbedingungen waren:
Einsatzstoff: TDA (Isomerengemisch aus 2,4- und 2,6-TDA im Gewichtsverhältnis von 80:20) gelöst in Dioxan (25 gew.-%ige Lösung), Temperatur = 170 °C, Wasserstoffdruck = 140 bar,
Katalysatormenge = 20 mg pro ml Einsatzstoff. Die Reaktionen wurden in einem Autoklaven durchgeführt.

Der Umsatz und die Selektivität wurden per GC-Analytik (GC-Säule: RTX^{®} Amin, Länge = 30 m, Innendurchmesser = 0,25 mm, Filmdicke = 0,5 µm) bestimmt und in Flächen-% angegeben. Tabelle 1 zeigt die erzielten Ergebnisse nach einer Reaktionszeit von 180 Minuten.

**Tabelle 1**

| **Metallgehalt [Gew.%]** | **Metall** | **TDA Umsatz [Flächen-%]** | **MDACH Selektivität [Flächen-%]** |
|---|---|---|---|
| **14,8** | Mo | 3,51 | 53,01 |
| **7,0** | Co | 2,66 | 41,59 |
| **10,3** | Ni | 3,78 | 47,32 |
| **9,9** | Pd | 82,48 | 1,66 |
| **5,0** | Pt | 9,00 | 57,80 |
| **5,0** | Rh | 99,21 | 33,45 |
| **12,1** | Ru | 93,56 | 85,87 |
| **5,0** | Ir | 16,36 | 13,09 |

Ruthenium zeigte als katalytisch aktives Metall mit 85,87% die höchste Selektivität bei hohem Umsatz (93,56%).

Die Katalysatorherstellung wird exemplarisch anhand des Co-Katalysators beschrieben. Alle weiteren Katalysatoren werden analog mit der dementsprechenden Metallsalzlösung hergestellt.

10 g Aluminiumoxid (NorPro nr. 2009850151) werden in einer Schale vorgelegt. Danach wird 5,49 g Cobalt(II)nitrathexahydrat in einem Messzylinder abgewogen und mit 7,6 ml destilliertem Wasser verdünnt. Die Lösung wird geviertelt und das Trägermaterial in der Schale in vier Schritten getränkt. Zwischen den Tränkschritten wird das Material mit einem Spatel homogenisiert. Das Pulver wird 16 h bei 120 °C getrocknet und im Anschluss 4 h bei 400 °C kalziniert. Der erhaltene Katalysator wird dann 2h bei 400 °C mit 4l/h H₂ und 40 l N₂/h reduziert und anschließend mit 5% Luft und 95 % N₂ 2h bei Raumtemperatur passiviert.

### Beispiel 2

Das katalytisch aktive Metall Ruthenium wurde auf verschiedenen Trägermaterialien in der Hydrierung von Toluoldiamin (TDA) zu Methyldiaminocyclohexan (MDACH) getestet und der Umsatz und die Selektivität gemessen.

Die Herstellung der eingesetzten Katalysatoren ist im Folgenden exemplarisch für einen erfindungsgemäßen Ru-Katalysator auf Zirkoniumoxid als Trägermaterial beschrieben. Die anderen in Tabelle 2 genannten Katalysatoren wurden entsprechend hergestellt:

30,51 g Ru(III)nitrosylnitratlösung (Fa. Heraeus) werden in einem Messzylinder vorgelegt und auf ein Gesamtvolumen von 37,5 ml mit VE-Wasser aufgefüllt. Danach werden 50 g Zirkoniumoxidpulver (D9-89, BASF, BET-Oberfläche: 78 m²/g, Porenvolumen: 0,84 ml/g, Porenvolumenverteilung: 68% Makroporen, 32% Mesoporen) in einer Keramikschale vorgelegt, die Lösung zugegeben und homogen gemischt. Anschließend wird das Pulver bei 120 °C im Umlufttrockenschrank für 16 h getrocknet und 2 h bei 200 °C unter Luft kalziniert. Dann wird das Pulver im Drehrohrofen erst 20 min mit 40 l/h N₂ gespült und danach innerhalb von 90 min reduziert (3 l/h Wasserstoff und 53 l/h Stickstoff). Nach Abkühlen auf Raumtemperatur wird der Wasserstoff abgestellt und mit ca. 60 l/h Stickstoff gespült. Zur Passivierung werden zunächst 60 l/h Stickstoff und 1 l/h Luft zugegeben und danach die Luftmenge langsam auf 10 l/h erhöht (0l/h Stickstoff). Dabei muss darauf geachtet werden, dass sich der Katalysator nicht über 35 °C erwärmt. Die so hergestellte Aktivmasse beträgt 10 Gew.-% Ru und 90 Gew.-% ZrO₂.

Der Katalysator weist folgende Charakteristika auf: Stampfdichte beträgt 1,13 kg/L, das Porenvolumen (Hg-Porosimetrie) beträgt 0,32 ml/g, die BET-Oberfläche beträgt 75 m²/g, die Porenverteilung ist wie folgt: 0% Mesoporen (2-50 nm), 100% Makroporen (>50 nm).

Die Reaktionsbedingungen für die Hydrierungsversuche waren: Einsatzstoff: TDA (Isomerengemisch aus 2,4- und 2,6-TDA im Gewichtsverhältnis von 80:20) gelöst in Dioxan (25 gew.-%ige Lösung), Temperatur = 170 °C, Wasserstoffdruck = 140 bar, Katalysatormenge = 20 mg pro mL Einsatzstoff. Die Reaktionen wurden in einem Autoklaven durchgeführt.

Der Umsatz und die Selektivität wurden per GC-Analytik (GC-Säule: RTX^{®} Amin, Länge = 30 m, Innendurchmesser = 0,25 mm, Filmdicke = 0,5 µm) bestimmt und in Flächen-% angegeben. Tabelle 2 zeigt die erzielten Ergebnisse nach einer Reaktionszeit von 180 Minuten.

**Tabelle 2**

| **Trägermaterial** | **Ru-Gehalt [Gew.-%]** | **TDA Umsatz [Flächen-%]** | **MDACH Selektivität [Flächen-%]** |
|---|---|---|---|
| **α-Al₂0₃** | 10,0 | 15,93 | 75,88 |
| **γ-Al₂0₃** | 10,2 | 80,37 | 83,75 |
| **γ-, θ-, δ-Al₂0₃** | 12,1 | 82,98 | 82,16 |
| **Boehmite** | 11,6 | 78,40 | 77,34 |
| **pseudo-Boehmite** | 10,4 | 57,78 | 78,25 |
| **Aktivkohle** | 10,0 | 59,66 | 35,11 |
| **Graphit** | 10,0 | 66,19 | 45,95 |
| **La₂O₃** | 9,7 | 59,68 | 56,23 |
| **SiO₂** | 10,0 | 45,11 | 65,99 |
| **TiO₂** | 10,0 | 16,80 | 67,42 |
| **Faujasite** | 10,0 | 3,26 | 58,06 |
| **Cr₂O₃** | 8,9 | 62,02 | 57,49 |
| **HA (Hydroxyapatit)** | 11,6 | 23,05 | 72,50 |
| **ZrO₂ (Gemisch aus monoklin, tetragonal)** | 10,0 | 40,06 | 85,87 |

Der erfindungsgemäße Katalysator enthaltend Ruthenium auf Zirkoniumoxid als Trägermaterial zeigte eine exzellente Selektivität bezüglich des gewünschten Produktes.

Die nachfolgend beschriebenen Hydrierungen gemäß den Beispielen 3 und 4 wurden in einem Rohrreaktor (Innendurchmesser 12 mm, Länge 140 cm) durchgeführt. Der Reaktor wurde dabei mit Umlauf betrieben, d.h. der Austrag wurde teilweise auf den Reaktor zurückgeführt, so dass eine Leerrohrgeschwindigkeit von 30 bis 60 m/h im Reaktor vorlag.

Die Hydrierung wurde mit reinem Wasserstoff durchgeführt. Der Zulauf wurde so gewählt, dass die Katalysatorbelastung im Reaktor (kg(TDA-Lösung)/(L(Katalysator)·h) den angegeben Wert erreicht. Der Wasserstoff wurde druckgeregelt bei dem angegebenen Druck zugeführt. Die Reaktionstemperaturen sind ebenfalls angegeben.

### Beispiel 3 (Vergleich)

In diesem Vergleichsbeispiel wurde die Deaktivierungsneigung eines Katalysators, der 1 Gew.-% Ruthenium auf einem gamma-Aluminiumoxid-Trägermaterial enthält in der Hydrierung von Toluoldiamin (TDA) zu Methyldiaminocyclohexan (MDACH) getestet und der Umsatz und die Selektivität gemessen. Die Reaktionsbedingungen waren:
Einsatzstoff: TDA (Isomerengemisch aus 2,4- und 2,6-TDA im Gewichtsverhältnis von 80:20) gelöst in Dioxan (25 gew.-%ige Lösung), Temperatur = 170 °C, Wasserstoffdruck = 190 bar, Katalysatorbelastung = 0,5 kg_{Einsatzstoff}·L_{Katalysator}⁻¹·h⁻¹, Leerrohrgeschwindigkeit 44 m/h

Der Umsatz und die Selektivität wurden per GC-Analytik (GC-Säule: RTX^{®} Amin, Länge = 30 m, Innendurchmesser = 0,25 mm, Filmdicke = 0,5 µm) bestimmt und in Flächen-% angegeben. Tabelle 3 zeigt die erzielten Ergebnisse in Abhängigkeit von der Reaktionszeit.

**Tabelle 3**

| **Zeit [h]** | **TDA Umsatz [Flächen-%]** | **MDACH Selektivität [Flächen-%]** |
|---|---|---|
| **22** | 91,5 | 79,9 |
| **41** | 88,4 | 80,8 |
| **65** | 85,8 | 79,9 |
| **185** | 79,3 | 78,8 |
| **209** | 76,8 | 79,0 |
| **233** | 74,9 | 79,2 |
| **256** | 75,4 | 77,9 |
| **280** | 74,1 | 77,2 |

Das Vergleichsbeispiel 3 zeigt, dass der TDA-Umsatz nach 280 Stunden von 91,5% (nach 22 Stunden) auf 74,1 % (nach 280 Stunden) abgesunken ist. Gleichzeitig ging die MDACH-Selektivität von 79,9% auf 77,2% zurück.

### Beispiel 4

### Herstellung Festbettkatalysator 1 Gew.-% Ru auf ZrO₂

238 g ZrO₂ Stränge (Ø 3 mm, SZ 31108 der Firma NorPro, BET-Oberfläche: 73 m²/g, Porenvolumen: 0,30 ml/g, Porenvolumenverteilung: 6% Makroporen, 94% Mesoporen) werden in einer Tränktrommel mit 19,81 g Ru(III)nitrosylnitratlösung (15.95 Gew% Ru(III)nitrosylnitrat (Fa Heraeus) in verdünnter Salpetersäure), verdünnt mit 35 ml VE-Wasser besprüht. Danach werden die Extrudate bei 120 °C für 16 h im Umlufttrockenschrank getrocknet und anschließend im Muffelofen für 2 h bei 180 °C kalziniert. Der Katalysator wird dann für 2 h bei 200 °C (4 l/h H₂; 40 l/h N₂) erst reduziert und mit einem Gemisch aus 10 Vol.% Luft und 90 Vol.% N₂ für 1 h bei Raumtemperatur passiviert. Die so hergestellte Aktivmasse enthält 1 Gew.-% Ru und 99 Gew.-% Zirkoniumoxid.

Der Katalysator weist folgende Charakteristika auf: Eine BET-Oberfläche von 81 m²/g, eine Stampfdichte von 1,2 kg/L, ein Porenvolumen von 0,24 mL/g (bestimmt durch Hg-Porosimetrie).

### Beispiel 5

In diesem erfindungsgemäßen Beispiel wurde die Reaktion gemäß Vergleichsbeispiel 3 durchgeführt, wobei der erfindungsgemäße Katalysator enthaltend 1 Gew.-% Ruthenium auf einem Zirkoniumoxid-Trägermaterial eingesetzt wurde, dessen Herstellung in Beispiel 2 exemplarisch gezeigt wird. Die Reaktionsbedingungen waren:
Einsatzstoff: TDA (Isomerengemisch aus 2,4- und 2,6-TDA im Gewichtsverhältnis von 80:20) gelöst in Dioxan (25 gew.-%ige Lösung), Temperatur = 170 °C, Wasserstoffdruck = 190 bar, Katalysatorbelastung = 0,1 1 kg_{Einsatzstoff}·L_{Katalysator}⁻¹·h⁻¹Leerrohrgeschwindigkeit 44 m/h.

Der Umsatz und die Selektivität wurden per GC-Analytik (GC-Säule: RTX^{®} Amin, Länge = 30 m, Innendurchmesser = 0,25 mm, Filmdicke = 0,5 µm) bestimmt und in Flächen-% angegeben. Tabelle 3 zeigt die erzielten Ergebnisse in Abhängigkeit von der Reaktionszeit.

**Tabelle 4:**

| **Zeit [h]** | **TDA Umsatz [Flächen-%]** | **MDACH Selektivität [Flächen-%]** |
|---|---|---|
| **18,0** | 97,9 | 53,2 |
| **26,0** | 97,6 | 61,0 |
| **42,0** | 96,6 | 73,3 |
| **73,0** | 96,8 | 69,2 |
| **146,0** | 95,5 | 76,1 |
| **170,0** | 95,3 | 76,0 |
| **193,5** | 94,9 | 76,3 |
| **218,5** | 94,6 | 76,7 |
| **242,0** | 94,1 | 76,1 |
| **330,0** | 88,2 | 74,5 |
| **354,0** | 89,5 | 71,8 |
| **380,5** | 94,4 | 75,4 |
| **402,0** | 94,8 | 75,4 |
| **475,0** | 95,6 | 75,4 |
| **498,0** | 94,9 | 78,0 |
| **522,5** | 95,4 | 75,8 |
| **572,5** | 95,6 | 75,5 |
| **650,0** | 95,0 | 76,5 |
| **672,0** | 95,5 | 75,8 |
| **696,5** | 95,6 | 75,5 |
| **720,0** | 95,5 | 75,5 |
| **744,0** | 95,4 | 76,1 |
| **818,0** | 94,8 | 78,1 |
| **840,0** | 95,2 | 75,8 |
| **864,0** | 95,2 | 75,8 |
| **890,0** | 95,2 | 75,9 |

Das Beispiel zeigt, dass der TDA-Umsatz nach 242 Stunden 94,1 %, nach 890 Stunden 95,2% beträgt, wobei die MDACH-Selektivität bei 242 Stunden und 890 Stunden praktisch unverändert hoch ist.

## Patentansprüche

1. Verfahren zur Hydrierung aromatischer Verbindungen mit Wasserstoff in Gegenwart eines Katalysators, **dadurch gekennzeichnet, dass** der Katalysator Ruthenium auf einem Zirkoniumoxid-Trägermaterial enthält.

2. Verfahren nach Anspruch 1, **dadurch gekennzeichnet, dass** die aromatische Verbindung ausgewählt ist aus der Gruppe bestehend aus Anilin, 2,4-Diaminotoluol, 2,6-Diaminotoluol, 2,3-Diaminotoluol, 2,5-Diaminotoluol, 3,4-Diaminotoluol, 3,5-Diaminotoluol, o-Phenylendiamin, m-Phenylendiamin, p-Phenylendiamin, m-Xylendiamin (MXDA), Bis-(4-amino-3-methylphenyl)-methan, Bis-(4-amino-3,5-dimethylphenyl)-methan und Mischungen davon.

3. Verfahren nach Anspruch 2 **dadurch gekennzeichnet, dass** die aromatische Verbindung ein Gemisch aus 2,4- und 2,6-Diaminotoluol ist, wobei das 2,4-Diaminotoluol in einem Anteil von 70 bis 90% und das 2,6-Diaminotoluol in einem Anteil von 10-30% vorliegt.

4. Verfahren nach einem der Ansprüche 1 bis 3, **dadurch gekennzeichnet, dass** das Verfahren kontinuierlich durchgeführt wird.

5. Verfahren nach einem der Ansprüche 1 bis 4, **dadurch gekennzeichnet, dass** das Verfahren bei einer Temperatur von 50 bis 220 °C durchgeführt wird.

6. Verfahren nach einem der Ansprüche 1 bis 5, **dadurch gekennzeichnet, dass** das Verfahren bei einem Wasserstoffdruck von 100 bis 300 bar durchgeführt wird.

7. Verfahren nach einem der Ansprüche 1 bis 6, **dadurch gekennzeichnet, dass** der Katalysator Ruthenium in einer Menge von 0,05 bis 15 Gew.-%, bezogen auf den gesamten Katalysator, enthält.

8. Verfahren nach einem der Ansprüche 1 bis 7, **dadurch gekennzeichnet, dass** das Zirkonoxid-Trägermaterial in monokliner, tetragonaler, kubischer, amorpher Phase oder einer Mischphase dieser Modifikationen vorliegt.

9. Verfahren nach einem der Ansprüche 1 bis 8, **dadurch gekennzeichnet, dass** das Zirkoniumoxid-Trägermaterial in monokliner, tetragonaler oder einer Mischphase dieser Modifikationen vorliegt.

10. Verfahren nach einem der Ansprüche 1 bis 9, **dadurch gekennzeichnet, dass** das Zirkonoxid-Trägermaterial eine BET-Oberfläche von 30 bis 300 m²/g, bevorzugt 50 bis 90 m²/g, ein Porenvolumen von 0,1 bis 1 cm³/g, bevorzugt 0,1 bis 0,9 cm³/g, und eine Stampfdichte von 500 bis 2000 kg/m³, bevorzugt 700 bis 1750 kg/m³, aufweist.

11. Verfahren nach einem der Ansprüche 1 bis 10, **dadurch gekennzeichnet, dass** der Katalysator eine BET-Oberfläche von 30 bis 300 m²/g, bevorzugt 50 bis 90 m²/g, ein Porenvolumen von 0,1 bis 1 cm³/g, bevorzugt 0,1 bis 0,9 cm³/g, und eine Stampfdichte von 500 bis 2000 kg/m³, bevorzugt 700 bis 1750 kg/m³, aufweist.

12. Verfahren nach einem der Ansprüche 1 bis 11 **dadurch gekennzeichnet, dass** das Zirkoniumoxid-Trägermaterial des im Festbett eingesetzten Katalysators eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

13. Verfahren nach einem der Ansprüche 1 bis 12 **dadurch gekennzeichnet, dass** der im Festbett eingesetzte Katalysator eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

14. Verfahren nach einem der Ansprüche 1 bis 13 **dadurch gekennzeichnet, dass** das Zirkoniumoxid-Trägermaterial des in Suspension eingesetzten Katalysators eine Porengrößenverteilung aufweist, bei der mehr als 40% der vorliegenden Poren durch Makroporen mit einem Durchmesser von >50 nm, und der Rest zu 100% durch Mesoporen mit einem Durchmesser von 2 nm bis 50 nm gebildet werden.

15. Verfahren nach einem der Ansprüche 1 bis 14 **dadurch gekennzeichnet, dass** der in Suspension eingesetzte Katalysator eine Porengrößenverteilung aufweist, bei der mehr als 40% der vorliegenden Poren durch Makroporen mit einem Durchmesser von >50 nm, und der Rest zu 100% durch Mesoporen mit einem Durchmesser von 2 nm bis 50 nm gebildet werden.

16. Verfahren nach einem der Ansprüche 1 bis 15, **dadurch gekennzeichnet, dass** die Hydrierung in einem organischen Lösungsmittel erfolgt.

17. Verwendung eines Katalysators enthaltend Ruthenium auf einem Zirkoniumoxid-Trägermaterial zur Hydrierung aromatischer Verbindungen.
